# EUROPÄISCHE PATENTSCHRIFT

(11) **EP 2 008 680 B1**
(45) Veröffentlichungstag und Bekanntmachung des Hinweises auf die Patenterteilung: **09.02.2011**
(21) Anmeldenummer: 08011402.8
(22) Anmeldetag: 24.06.2008
(51) Int. Cl.: A61M 16/04, A61F 13/12

(54) **Duschschutz für Tracheotomierte und/oder Laryngektomierte**
Shower protection for tracheotomy and/or laryngitis patients
Protection de douche pour patient ayant subi une trachéotomie et/ou une laryngectomie

(30) Priorität: 28.06.2007 DE 102007030092; 28.06.2007 DE 202007009105 U
(43) Veröffentlichungstag der Anmeldung: 31.12.2008
(73) Patentinhaber: Andreas Fahl Medizintechnik-Vertrieb GmbH, 51149 Köln (DE)
(72) Erfinder: Fahl, Andreas, 51107 Köln (DE)
(74) Vertreter: Maxton Langmaack & Partner

(56) Entgegenhaltungen:
- EP-A- 0 370 962
- US-A- 3 464 410
- US-A- 5 487 382

## Beschreibung

Die vorliegende Erfindung betrifft einen Duschschutz für Tracheotomierte und/oder Laryngektomierte gemäß dem Oberbegriff des Anspruchs 1.

Bei operativen Eingriffen im oberen Atemtrakt kann das Anlegen einer künstlichen Atemöffnung (Tracheostoma) der Luftröhre notwendig sein, damit unter Umgehung von Mundraum und Kehlkopf Luft direkt in die Lunge eingeatmet werden kann. Bei Laryngektomierten, also kehlkopflosen Personen, bei welchen der Kehlkopf operativ entfernt wurde, muss das Tracheostoma dauerhaft offen gehalten und stabilisiert werden, wozu insbesondere Trachealkanülen in das Tracheostoma eingesetzt werden. Aber auch der Einsatz sogenannter "Tracheostoma-Button" ist hier möglich - insbesondere für Patienten, die keine Trachealkanüle mehr benötigen.

Des Weiteren können in das Tracheostoma auch sogenannten "Shunt-Ventile" eingesetzt werden, welche eine Wiederherstellung der Stimme ermöglichen. Schließlich können in das Tracheostoma auch Filtersysteme eingesetzt werden, sowohl bei Tracheotomierten als auch bei Laryngektomierten. Derartige Filtersysteme bestehen entweder aus einem Pflaster mit einem eingesetzten Filter oder aus einer üblicherweise selbstklebenden Basisplatte - in aller Regel aus Kunststoff gebildet, in welche Filter unterschiedlichster Art einsetzbar sind.

Im täglichen Leben müssen Laryngektomierte und/oder Tracheotomierte, welche zusammen als Halsatmer bezeichnet werden können, darauf achten, dass die bei diesen eingesetzten Hilfsmittel vor Außeneinflüssen geschützt werden. Hierzu zählt insbesondere das mögliche Eindringen von Feuchtigkeit über eingesetzte Trächeaikanülen, Filtern etc.. Daher ist es für Halsatmer notwendig, die am Hals befindliche Körperöffnung beim Haare waschen, Duschen oder Baden zu schützen.

Aus dem Stand der Technik bekannt sind hierzu verschiedenste Vorrichtungen, welche unmittelbar an einen Tracheostoma - Pflaster oder einer Trachealkanüle angeordnet werden können. So offenbart die DE 20 2005 008 069 U1 einen Duschschutz zum Duschen für Patienten mit einem Tracheostoma zum Schutz vor eindringendem Wasser in das Tracheostoma, wobei der Duschaufsatz mit einer mit einer durch einen Verschlussdeckel verschließbaren Wasserfalle mit Rinne für Kriechwasser ausgebildet ist und durch eine im Duschaufsatz eingearbeitete Ringnut die Montage und Demontage eines Filtergehäuses ermöglicht, sowie durch den am Filtergehäuse angebrachten Arretierungsring die Kombination mit einem Tracheostomapflaster oder einer Trachealkanüle erlaubt.

Nachteil derartiger Vorrichtungen ist, dass diese in aller Regel nur auf spezifische Produkte passend anbringbar sind, so dass bei Verwendung unterschiedlichster Produkte durch den Halsatmer auch unterschiedliche Duschschutz-Vorrichtungen im Sinne der DE 20 2005 008 069 U1 benötigt werden.

Aus der US 3 464 410 A ist ein Stoma-Schutzlätzchen bekannt, das drei übereinander liegende Blätter aufweist, die an ihren Kanten mit Hilfe eines Einfassbands aneinander befestigt sind.

Es ist daher Aufgabe der Erfindung, einen Duschschutz zur Verfügung zu stellen, welcher unabhängig von dem eingesetzten Hilfsmittel dem Halsatmer insbesondere das Duschen, Waschen einschließlich Haare waschen oder Baden einfach ermöglicht.

Zur Lösung dieser Aufgabe wird ein Duschschutz für Tracheotomierte und/oder Laryngektomierte gemäß Anspruch 1 vorgeschlagen, wobei zumindest am unteren Rand über zumindest eine Teillänge desselben ein Lippenelement angeordnet ist, um bei Verwendung im unteren Halsbereich eine Dichtung zu erzielen. Der erfindungsgemäße Duschschutz zeichnet sich daher dadurch aus, dass dieser aufgrund des beabstandet vom Hals angeordneten Abdeckungsbereiches die unterschiedlichen möglichen Hilfsmittel, welche bei einem Halsatmer zur Verwendung kommen, abdeckt, wobei eine Befestigung des erfindungsgemäßen Duschschutzes einfach am Hals des Halsatmers erfolgt. Der Abdeckungsbereich, der halbnapfartig ausgebildet sein kann, legt sich dabei mit seinem oberen Rand unterhalb des Kinns an, so dass hier ein gut dichtender Sitz gewährleistet ist. Durch die Vorsehung eines Lippenelementes am unteren Rand des Abdeckbereiches ist vorteilhaft sicher gestellt, dass auch im von seiner Oberfläche her ungleichmäßiger ausgebildeten unteren Halsbereich eine gute Dichtung gegen Waschflüssigkeiten, Wasser etc. gegeben ist. In einer alternativen Ausführungsform kann vorgesehen sein, dass auch am oberen Rand über zumindest eine Teillänge desselben ebenfalls ein solches Lippenelement angeordnet ist. Das Lippenelement selbst kann im Querschnitt geradlinig, keilförmig, abgerundet bzw. gewölbt bzw. in jedweder sonstigen Ausgestaltung ausgebildet sein.

In einer bevorzugten Ausführungsform kann dabei das Lippenelement nicht nur im Bereich des unteren Abdeckungsbereiches, sondern auch in einem Übergangsbereich vom Abdeckungsbereich zum Befestigungsbereich angeordnet sein.

Vorteilhafterweise ist das Lippenelement elastisch ausgebildet. Hierdurch kann sich dieses an die ungleichmäßige Oberfläche im unteren Halsbereich eng anlegen. Zudem ist es vorzugsweise flexibel ausgebildet, wodurch weiterhin die gute Anlage des Abdeckungsbereiches am Hals sichergestellt ist.

In einer weiter bevorzugten Ausführungsform steht das Lippenelement winklig vom unteren Rand ab. Dabei ist weiter bevorzugt vorgesehen, dass das Lippenelement proximal, d.h. zum Körper hin, ausgerichtet ist. Vorzugsweise liegt die Abwinklung in einem Bereich von etwa 10° bis etwa 60°, weiter bevorzugt in einem Bereich von etwa 25° bis etwa 50° bezogen auf eine senkrecht zum unteren Rand verlaufende Ebene, welche den unteren Rand einschließt.

Weiter bevorzugt erstreckt sich das Lippenelement über die gesamte Länge des unteren Randes, und alternativ zusätzlich auch über die gesamte Länge des oberen Randes des Abdeckungsbereiches. Wie bereits weiter oben angesprochen, kann hier auch bevorzugt eine Erstreckung im Bereich des unteren Randes in den Übergangsbereich zwischen Abdeckungsbereich und Befestigungsbereich vorgesehen sein.

Vorteilhafterweise ist im Übergangsbereich vom Abdeckungsbereich zum Befestigungsbereich das Lippenelement sich verbreiternd ausgebildet. Hierdurch wird auch in dem kritischen Seitenbereich des Abdeckungsbereiches eine gute Anlage und damit ein guter Schutz vor eindringenden Flüssigkeiten bei dem erfindungsgemäßen Duschschutz erzielt.

Weiter vorteilhaft umfasst der Befestigungsbereich mindestens zwei Halteelemente, welche beidseitig und gegenüberliegend an dem Abdeckungsbereich angeordnet sind. Vorteilhafterweise sind die Halteelemente bandförmig ausgebildet. Die Halteelemente können dabei auf unterschiedlichste Art und Weise miteinander verbindbar ausgebildet sein, beispielsweise durch Klettverschlüsse, Knoten, Schnallen oder ähnlichem. Bevorzugt ist jedoch ein Knopfverschluss vorgesehen, wobei durch Vorsehung von mindestens einer Öffnung das Knopfverschlusselement, welches in einem der beiden Haltelement vorgesehen ist, durch die in dem anderen Halteelement vorgesehene Öffnung zur Schließung führbar ist. Um eine Verstellbarkeit im Hinblick auf unterschiedliche Halsstärken vorzusehen, können dabei auch in dem einen oder beiden Halteelementen mehrere Öffnungen vorgesehen sein, in welche auch das Knopfverschlusselement austauschbar einführbar ist. Hierdurch kann eine optimale Anpassung an einen Hals eines Halsatmers vorgenommen werden. Es sind selbstverständlich noch eine Reihe alternativer Ausführungsformen im Hinblick auf den Befestigungsbereich des erfindungsgemäßen Duschschutzes denkbar. So kann beispielsweise an lediglich einer Seite des Abdeckungsbereiches ein bandförmiges Halteelement vorgesehen sein, welches einfach in ein auf der gegenüberliegenden Seite des Abdeckelementes angeordnetes Verschlusselement einführbar ist. Darüber hinaus kann auch vorgesehen sein, dass zur Erzielung eines festeren und sicheren Sitzes auf mindestens einer Seite des Abdeckungsbereiches mindestens zwei Halteelemente vorgesehen sind, welche beispielsweise bei einer bandförmigen Ausbildung zusammen und/oder getrennt um den Hals des Halsatmers führbar sind zur Befestigung des erfindungsgemäßen Duschschutzes.

In einer weiter bevorzugten Ausführungsform weist der Abdeckungsbereich mindestens ein Verstärkungselement auf. Das Verstärkungselement dient dabei der Formstabilität des Abdeckungsbereiches, so das dieses stets beabstandet vom Hals des Halsatmers angeordnet ist. Durch das Verstärkungselement ist ein Zusammenfallen des Abdeckungsbereiches sicher vermieden, wodurch dem Halsatmer auch ein Atmen durch das Tracheostoma nicht mehr möglich wäre. Das Verstärkungselement kann dabei sowohl außerhalb als auch innerhalb des Abdeckungsbereiches angeordnet sein, und auch aus unterschiedlichen Materialien gebildet sein. Insbesondere kann es auch als Einsatz ausgebildet sein, sodass dann der erfindungsgemäße Duschschutz mindestens zweiteilig ausgebildet ist. Besonders bevorzugt ist aber das Verstärkungselement einteilig, weiter bevorzugt auch einstoffig, mit dem Abdeckungsbereich verbunden bzw. ausgebildet. Mögliche nicht einstoffige Ausführungsformen sind beispielsweise das Vorsehen von Metallplättchen bzw. -brücken im Bereich von Öffnungen im Abdeckungsbereich, welche ein Atmen für den Halsatmer ermöglichen. Es können aber auch beispielsweise diese Öffnungen mit metallischen oder Kunststoffnieten versehen sein, sodass hierdurch eine Stabilisierung des Abdeckungsbereiches erzielt wird.

In einer bevorzugten Ausführungsform weist das Verstärkungselement mindestens eine erste Verstrebung auf. Vorteilhafterweise weist das Verstärkungselement mindestens zwei, noch weiter bevorzugt mindestens drei derartige erste Verstrebungen auf.

Vorteilhafterweise ist das Verstärkungselement im Inneren, d.h. in dem vom Abdeckungsbereich gebildeten, als halbnapfartig anzusprechenden gebildeten Raum des Abdeckungsbereiches angeordnet.

Weiter bevorzugt sind die ersten Verstrebungen etwa parallel zueinander ausgebildet. In einer weiter bevorzugten Ausführungsform gehen die ersten Verstrebungen von einem Bodenbereich des Abdeckungsbereich in einen Wandbereich des selben über. Der Bodenbereich kann dabei bevorzugt mindestens eine Öffnung, bevorzugt mehrere Öffnungen aufweisen, welche dem Halsatmer ein Atmen über das Tracheostoma ermöglichen.

In einer weiter bevorzugten Ausführungsform des erfindungsgemäßen Duschschutzes ist die erste Verstrebung im Übergang zwischen Bodenbereich und Wandbereich verdickt ausgebildet. Durch diese Materialanhäufung im genannten Übergangsbereich wird die Stabilität des Abdeckungselementes, insbesondere dessen Formstabilität, weiter erhöht.

In einer weiter bevorzugten Ausführungsform des erfindungsgemäßen Duschschutzes weist das Verstärkungselement mindestens eine zweite, in etwa quer zu der ersten Verstrebung verlaufende zweite Verstrebung auf. Dabei kann auch vorgesehen sein, dass mehrere zweite Verstrebungen vorgesehen sind. Vorteilhafterweise kreuzt die zweite Verstrebung mindestens eine der ersten Verstrebungen. Sind mehrere erste Verstrebungen in etwa parallel zueinander angeordnet vorgesehen, so kreuzt die zweite Verstrebung zumindest die mittlere bzw. die mittig liegenden Verstrebungen, kann jedoch in einer bevorzugten Ausführungsform auch sämtliche erste Verstrebungen kreuzen. Dabei ist weiter bevorzugt die zweite Verstrebung derart ausgebildet, dass diese in ihrem mittleren Bereich, insbesondere im Bereich der Kreuzungen mit den ersten Verstrebungen, mit einer größeren Stärke (Höhe) als die ersten Verstrebungen ausgebildet ist. Weiter bevorzugt ist die zweite Verstrebung im Wandbereich des Abdeckungsbereiches angeordnet, kann alternativ jedoch auch im Bodenbereich oder aber sowohl im Boden als auch im Wandbereich [des Abdeckungsbereiches] angeordnet sein.

Diese und weitere Vorteile der vorliegenden Erfindung werden anhand der vorliegenden Figuren näher erläutert. Es zeigen:
- Fig. 1:: eine Seitenansicht eines erfindungsgemäßen Duschschutzes;
- Fig. 2:: eine Aufsicht auf den erfindungsgemäßen Duschschutz gemäß Fig. 1;
- Fig. 3:: eine Schnittansicht durch den Duschschutz gemäß Fig. 1 entlang eines Schnittes III-III;
- Fig.4:: eine zweite Ausführungsform eines erfindungsgemäßen Duschschutzes entsprechend einem Schnitt III-III der ersten Ausführungsform gemäß Fig. 1.

Zunächst sei darauf hingewiesen, dass die Erfindung nicht auf die in den Figuren gezeigten Merkmalskombinationen beschränkt ist. Vielmehr sind die jeweils in der Beschreibung einschließlich der Figurenbeschreibung offenbarten Merkmale mit denjenigen in den Figuren angegebenen Merkmalen kombinierbar. Insbesondere sei darauf hingewiesen, dass die in den Patentansprüchen aufgenommenen Bezugszeichen in keiner Weise den Schutzbereich der vorliegenden Erfindung beschränken sollen, sondern lediglich auf die in den Figuren gezeigten Ausführungsbeispiele verweisen. Insbesondere sei darauf hingewiesen, dass die in den Figuren gezeigte Ausbildung des Verstärkungselementes als auch die Anzahl der ersten und zweiten Verstrebungen des Verstärkungselementes den Gegenstand der vorliegenden Erfindung in keiner Weise beschränken, ebenso wie die konkrete Ausbildung des Befestigungsbereiches des erfindungsgemäßen Duschschutzes.

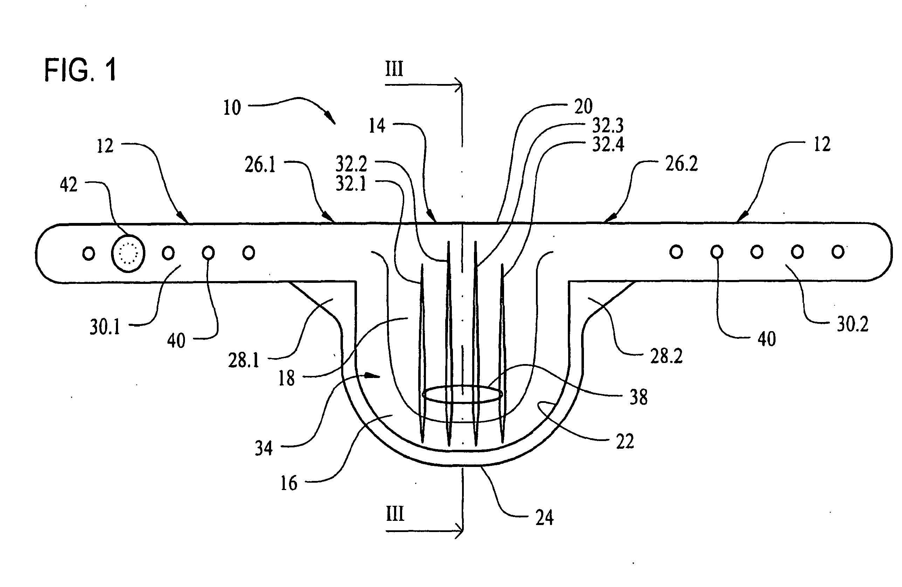

Fig. 1 bezeichnet einen insgesamt mit dem Bezugszeichen 10 bezeichneten Duschschutz mit einem Befestigungsbereich 12 und einem Abdeckungsbereich 14. Der Befestigungsbereich 12 ist dabei durch zwei bandförmige Halteelemente 30.1 und 30.2 bebildet, welche beidseitig des Abdeckungsbereiches 14 und gegenüberliegend angeordnet sind. Dabei weisen beide Halteelemente 30.1 und 30.2 jeweils fünf Öffnungen 40 auf, und im Halteelement 30.1 ist ein Knopfverschlusselement 42 austauschbar angeordnet. Über dieses Knopfverschlusselement 42 ist bei Durchführung durch eine Öffnung 40 des weiteren Halteelementes 30.2 der Duschschutz am Hals eines Halsatmers sicher befestigbar.

Der Abdeckungsbereich 14 geht in den Befestigungsbereich 12 über, wobei zwei Übergangsbereiche 26.1 und 26.2 gebildet sind. Der Abdeckungsbereich 14 weist dabei einen oberen Rand 20 auf, welcher bei der in der Fig. 1 gezeigten Ausführungsform bündig in die Übergangsbereiche 26.1 und 26.2 sowie die Halteelemente 30.1 und 30.2 ohne Versprung übergeht. Hier sind jedoch auch weitere alternative Ausbildungen möglich, beispielsweise können die Halteelemente 30.1 und 30.2 auch im Verhältnis zum oberen Rand 20 des Abdeckungsbereiches 14 versetzt tiefer beidseitig des Abdeckungsbereiches 14 angeordnet sein.

Des Weiteren weist der Abdeckungsbereich 14 einen unteren Rand 22 auf, an welchem über dessen gesamte Länge ein elastisches Lippenelement 24 angeordnet ist. Dieses Lippenelement 24 weist in den Übergangsbereichen 26.1 und 26.2 Verbreiterungen 28.1 und 28.2 auf. In den Verbreiterungen kann auch das Lippenelement mit einer größeren Stärke ausgeführt sein, bevorzugt ist es jedoch über seine gesamte Länge mit einer gleich bleibenden Stärke ausgebildet.

Im Übrigen kann der Übergang vom Abdeckungsbereich 14 und dessen unteren Rand 22 in den Befestigungsbereich 12 und die Halteelemente 30.1 und 30.2 nicht nur, wie in der Fig. 1 gezeigt, unter einer rechtwinkligen Anordnung erfolgen, hier sind insbesondere auch einerseits abgerundete Ausbildungen des unteren Randes 22 im Übergangsbereich 26.1 bzw. 26.2 möglich, und insbesondere auch ein stumpfwinkliger Verlauf des unteren Randes 22 bei Übergang in den unteren Randbereich der Halteelemente 30.1 und 30.2.

In einem Innenraum 34 des halbnapfartig ausgebildeten Abdeckungsbereiches 14 ist ein Verstärkungselement mit insgesamt vier ersten Verstrebungen 32.1 bis 32.4 angeordnet, welche ausgehend vom einem Bodenbereich 16 des Abdeckungsbereiches 14 in einen Wandbereich 18 des Abdeckungsbereiches 14 übergehend angeordnet sind. Dabei ist weiterhin eine zweite Verstrebung 38 im Wandbereich 18 vorgesehen, welche sich vollständig über die beiden mittleren ersten Verstrebungen 32.2 und 32.3 erstreckt, und im Bereich der äußeren ersten Verstrebungen 32.1 und 32.4 ausläuft. Dabei ist die zweite Verstrebung 38 zur Mitte hin verdickt ausgebildet, um eine größere Stabilität dem Verstärkungselement zu verleihen.

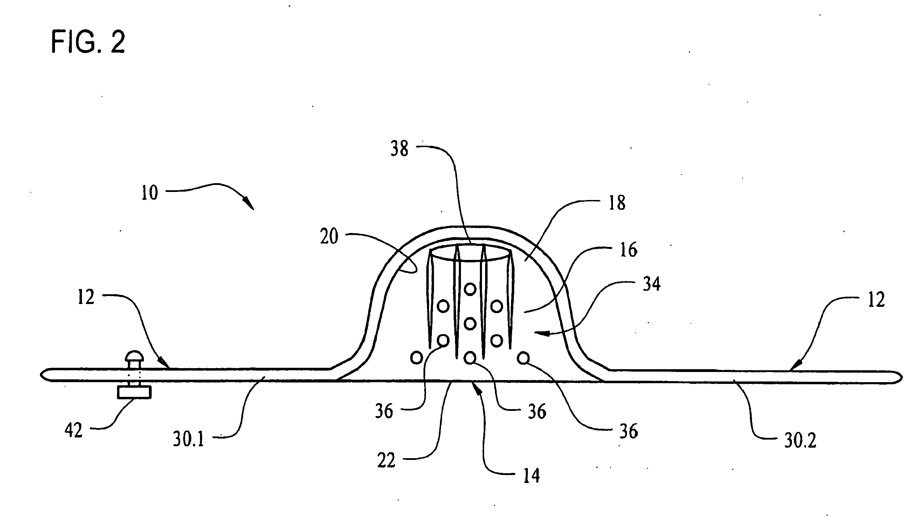

Fig. 2 zeigt nun den erfindungsgemäßen Duschschutz 10 gemäß Fig. 1 in einer Aufsicht. Hierbei sind insbesondere gut die im Bodenbereich 16 des Abdeckungsbereiches 14 angeordneten Öffnungen 36 zu erkennen, welche dem Halsatmer ein Atmen durch das Tracheostoma bei angelegtem Duschschutz 10 ermöglichen. Des Weiteren ist auch gut die Ausbildung des Knopfverschlusselementes 42 erkennbar, als auch die Anordnung des Verstärkungselementes mit den ersten Verstrebungen 32 und der zweiten Verstrebung 38 im Innenraum 34 des Abdeckungsbereiches 14.

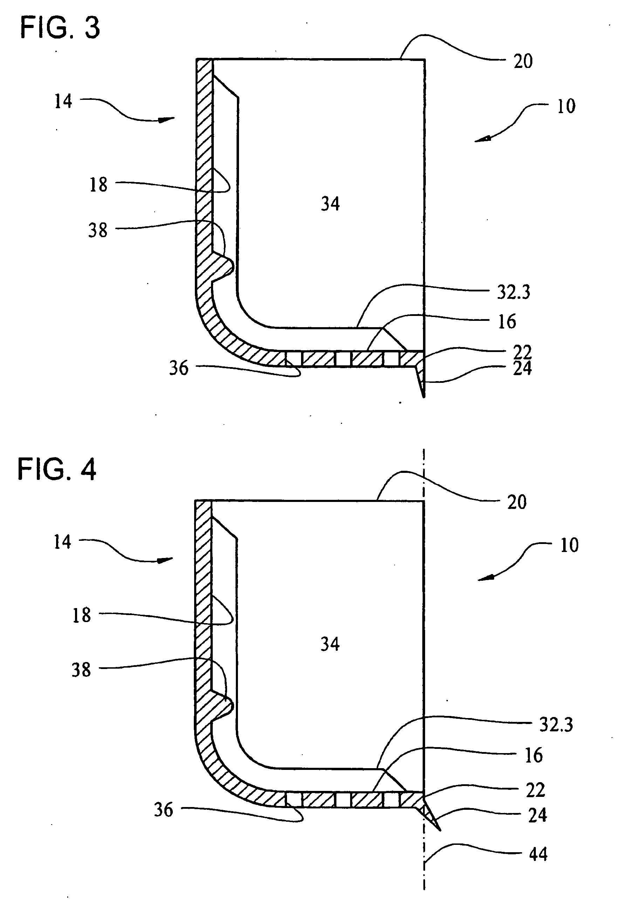

Fig. 3 zeigt nun den Duschschutz 10 gemäß Fig. 1 entlang eines Schnittes III-III. Besonders gut ist hierbei die Ausbildung der zweiten Verstrebung 38, angeordnet am Wandbereich 18 des Abdeckungsbereiches 14 zu erkennen, als auch eine Ausbildung derselben derart, dass diese eine Höhe bzw. Stärke aufweist, welche nicht über die Höhe der ersten Verstrebung 32.3 hinausgeht. Alternativ kann in einer bevorzugten Ausführungsform jedoch vorgesehen sein, dass die Höhe der ersten Verstrebung 38 gerade im Bereich der ersten Verstrebung 32.3 bzw. 32.2 über die Höhe dieser ersten Verstrebungen 32.2 und 32.3 hinausragt. Des Weiteren ist Fig. 3 gut zu entnehmen, dass das Lippenelement 24 am unteren Rand 22 angeordnet ist, und von diesem rechtwinklig absteht. Dabei ist das Lippenelement 24 mit einer leichten Materialverstärkung im Querschnitt etwa keilförmig ausgehend vom Anschlussbereich an den unteren Rand 22.2 und verjüngend zu dessen freiem Ende ausgebildet. Aber auch andere Ausbildungen des Lippenelementes 24 sind möglich.

Fig. 4 zeigt nun eine Alternative Ausführungsform zu dem Duschschutz 10 gemäß den Fig. 1 bis 3, wobei im Folgenden nur die Unterschiede hierzu angeführt werden. Das Lippenelement 24 ist dabei im Bereich des unteren Randes 24 proximal auf den Körper eines Halsatmers hin ausgerichtet und winklig ausgebildet in einem Winkel von etwa 30°, bezogen auf eine Ebene 44, welche senkrecht zum unteren Rand 22 verläuft und diesen unteren Rand 22 einschließt.

Durch die vorliegende Erfindung wird somit ein Duschschutz zur Verfügung gestellt, welcher aufgrund der Vorsehung eines spezifischen Lippenelementes gerade im unteren Halsbereich eine hervorragende Abdichtung bietet, und darüber hinaus durch die Vorsehung eines im Innenbereich des Abdeckungsbereiches angeordneten Verstärkungselementes nicht nur dessen einstückige bzw. einstoffige Ausbildung ermöglicht (sieht man von dem Knopfverschlusselement ab), sondern auch kostengünstig herstellbar und optisch gefällig ausbildbar ist.

## Patentansprüche

1. Duschschutz (10) für Tracheotomierte und/oder Laryngektomierte mit
- einem Befestigungsbereich (12) zur Befestigung des Duschschutzes (10) an einem Hals und
- einem Abdeckungsbereich (14) zur beabstandeten Abdeckung einer Körperöffnung mit einem oberen Rand (20) und einem unteren Rand (22),
**dadurch gekennzeichnet, dass** zumindest am unteren Rand (22) über zumindest eine Teillänge desselben ein Lippenelement (24) angeordnet ist, um bei Verwendung im unteren Halsbereich eine Dichtung zu erzielen

2. Duschschutz gemäß Anspruch 1, **dadurch gekennzeichnet, dass** das Lippenelement (24) elastisch ausgebildet ist.

3. Duschschutz gemäß einem der vorhergehenden Ansprüche, **dadurch gekennzeichnet, dass** das Lippenelement (24) vom unteren Rand (22) winklig absteht.

4. Duschschutz gemäß einem der vorhergehenden Ansprüche, **dadurch gekennzeichnet, dass** das Lippenelement (24) proximal ausgerichtet ist.

5. Duschschutz gemäß einem der vorhergehenden Ansprüche, **dadurch gekennzeichnet, dass** das Lippenelement (24) sich über die gesamte Länge des unteren Randes (22) erstreckt.

6. Duschschutz gemäß einem der vorhergehenden Ansprüche, **dadurch gekennzeichnet, dass** in einem Übergangsbereich (26.1, 26.2) vom Abdeckungsbereich (14) zum Befestigungsbereich (12) das Lippenelement (24) sich verbreiternd (28.1, 28.2) ausgebildet ist.

7. Duschschutz gemäß einem der vorhergehenden Ansprüche, **dadurch gekennzeichnet, dass** der Befestigungsbereich (12) mindestens zwei Halteelemente (30.1, 30.2), welche beidseitig und gegenüberliegend an dem Abdeckbereich (14) angeordnet sind, umfasst.

8. Duschschutz gemäß Anspruch 7, **dadurch gekennzeichnet, dass** die Halteelemente (30.1, 30.2) bandförmig ausgebildet sind.

9. Duschschutz gemäß einem der vorhergehenden Ansprüche, **dadurch gekennzeichnet, dass** der Abdeckungsbereich (14) mindestens ein Verstärkungselement aufweist.

10. Duschschutz gemäß Anspruche 9, **dadurch gekennzeichnet, dass** das Verstärkungselement im Inneren (34) des Abdeckungsbereiches (14) angeordnet ist.

11. Duschschutz gemäß einem der vorhergehenden Ansprüche, **dadurch gekennzeichnet, dass** im Bodenbereich (16) mindestens eine Öffnung (36) vorgesehen ist.

12. Duschschutz gemäß einem der vorhergehenden Ansprüche, **dadurch gekennzeichnet, dass** dieser einstückig ausgebildet ist.

13. Duschschutz gemäß einem der vorhergehenden Ansprüche, **dadurch gekennzeichnet, dass** dieser einstoffig ausgebildet ist.

## Claims

1. A shower shield (10) for tracheotomy and/or laryngectomy patients, having
- a securing region (12) for securing the shower shield (10) to a neck and
- a covering region (14) for the covering, at a distance, of a body opening, having an upper edge (20) and a lower edge (22),
**characterised in that** a lip member (24) is arranged at least at the lower edge (22), over at least a partial length thereof, in order to obtain a seal upon utilisation in the lower region of the neck.

2. A shower shield according to claim 1, **characterised in that** the lip member (24) is resilient.

3. A shower shield according to any one of the preceding claims, **characterised in that** the lip member (24) projects angularly from the lower edge (22).

4. A shower shield according to any one of the preceding claims, **characterised in that** the lip member (24) is directed proximally.

5. A shower shield according to any one of the preceding claims, **characterised in that** the lip member (24) extends over the entire length of the lower edge (22).

6. A shower shield according to any one of the preceding claims, **characterised in that** the lip member (24) widens (28.1, 28.2) in a transition region (26.1, 26.2) from the covering region (14) to the securing region (12).

7. A shower shield according to any one of the preceding claims, **characterised in that** the securing region (12) comprises at least two holding members (30.1, 30.2) arranged on either side of the covering region (14) so as to be opposite one another.

8. A shower shield according to claim 7, **characterised in that** the holding members (30.1, 30.2) are band-shaped.

9. A shower shield according to any one of the preceding claims, **characterised in that** the covering region (14) has at least one reinforcing member.

10. A shower shield according to claim 9, **characterised in that** the reinforcing member is arranged in the interior (34) of the covering region (14).

11. A shower shield according to any one of the preceding claims, **characterised in that** at least one opening (36) is provided in the bottom region (16).

12. A shower shield according to any one of the preceding claims, **characterised in that** the shower shield is single-piece.

13. A shower shield according to any one of the preceding claims, **characterised in that** the shower shield is made of one material.

## Revendications

1. Protection de douche (10) pour sujet trachéotomisé et/ou laryngectomisé, comportant
- une zone de fixation (12) pour fixer la protection de douche (10) au cou, et
- une zone de recouvrement (14), destinée à couvrir à distance un orifice du corps et munie d'un bord supérieur (20) et d'un bord inférieur (22),
**caractérisée en ce qu'**au moins au niveau du bord inférieur (22), sur au moins une partie de celui-ci, est agencé un élément de type lèvre (24), pour obtenir un système étanche en cas d'utilisation dans la région inférieure du cou.

2. Protection de douche selon la revendication 1, **caractérisée en ce que** l'élément de type lèvre (24) est élastique.

3. Protection de douche selon l'une quelconque des revendications précédentes, **caractérisée en ce que** l'élément de type lèvre (24) est en saillie angulaire sur le bord inférieur (22).

4. Protection de douche selon l'une quelconque des revendications précédentes, **caractérisée en ce que** l'élément de type lèvre (24) est orienté vers le côté proximal.

5. Protection de douche selon l'une quelconque des revendications précédentes, **caractérisée en ce que** l'élément de type lèvre (24) s'étend sur toute la longueur du bord inférieur (22).

6. Protection de douche selon l'une quelconque des revendications précédentes, **caractérisée en ce que** l'élément de type lèvre (24) est réalisé en s'élargissant (28.1, 28.2) dans une zone de transition (26.1, 26.2) menant de la zone de recouvrement (14) vers la zone de fixation (12).

7. Protection de douche selon l'une quelconque des revendications précédentes, **caractérisée en ce que** la zone de fixation (12) comporte au moins deux éléments de fixation (30.1, 30.2), qui sont disposés de part et d'autre et l'un en face de l'autre au niveau de la zone de recouvrement (14).

8. Protection de douche selon la revendication 7, **caractérisée en ce que** les éléments de fixation (30.1, 30.2) sont réalisés en forme de bandes.

9. Protection de douche selon l'une quelconque des revendications précédentes, **caractérisée en ce que** la zone de recouvrement (14) comporte au moins un élément de renfort.

10. Protection de douche selon la revendication 9, **caractérisée en ce que** l'élément de renfort est agencé à l'intérieur (34) de la zone de recouvrement (14).

11. Protection de douche selon l'une quelconque des revendications précédentes, **caractérisée en ce qu'**au moins un orifice (36) est prévu dans la zone de fond (16).

12. Protection de douche selon l'une quelconque des revendications précédentes, **caractérisée en ce que** celle-ci est réalisée d'un seul tenant.

13. Protection de douche selon l'une quelconque des revendications précédentes, **caractérisée en ce que** celle-ci est réalisée dans un seul matériau.
